Europäisches Patentamt

European Patent Office

Office européen des brevets

(11) Veröffentlichungsnummer: **0 411 443 A2**

# EUROPÄISCHE PATENTANMELDUNG

(21) Anmeldenummer: **90114180.4**

(22) Anmeldetag: **24.07.90**

(51) Int. Cl.5: **G01N 27/04, G01N 33/12**

(30) Priorität: **31.07.89 DE 3925331**

(43) Veröffentlichungstag der Anmeldung:
**06.02.91 Patentblatt 91/06**

(84) Benannte Vertragsstaaten:
**AT BE CH DE DK ES FR GB GR IT LI LU NL**

(71) Anmelder: **Thien, Gerhard**
**Stoffenerstrasse 12**
**D-8911 Ummendorf(DE)**

(72) Erfinder: **Thien, Gerhard**
**Stoffenerstrasse 12**
**D-8911 Ummendorf(DE)**

(74) Vertreter: **Bohnenberger, Johannes, Dr. et al**
**Meissner, Bolte & Partner**
**Widenmayerstrasse 48 48**
**D-8000 München 22(DE)**

(54) Fleischklassifizierungsgerät.

(57) Beim Klassifizieren von Hälften frisch geschlachteter Schweine muß insbesondere der Fleischanteil von Kotelettsträngen festgestellt werden. Es wird ein Fleischklassifizierungsgerät mit einer in ein zu untersuchendes Prüfstück einstechbaren Sonde und einer angekoppelten Auswerteinrichtung zum Feststellen und Anzeigen des Fleisch-/Fettanteils aufgezeigt. Die Sonde ist als Leitfähigkeitssonde ausgebildet und umfaßt einen Sondenkörper mit einem metallischen Trägerrohr, in dessen Oberfläche eine Elektrodenanordnung bündig eingelassen ist. Die Elektroden sind als Reihe isoliert zum Trägerkörper in diesem eingelassen und sind mit einer Auswerteinrichtung derart verbunden, daß bei eingestochener Leitfähigkeitssonde die Leitfähigkeitswerte zwischen allen Elektroden und dem Trägerrohr feststellbar und untereinander vergleichbar sind.

FIG.1

EP 0 411 443 A2

# FLEISCHKLASSIFIZIERUNGSGERÄT

Die Erfindung betrifft ein Fleischklassifizierungsgerät nach dem Oberbegriff des Patentanspruches 1, das zur Fleischklassifizierung frisch geschlachteter Schweinehälften, insbesondere zur Feststellung des Fleischanteils von Kotelettsträngen dient.

Frisch geschlachtete Schweinehälften müssen nach dem Schlachten klassifiziert werden, um das Fleisch dann entsprechend den gesetzlichen Bestimmungen mit einer Güteklasse bezeichnen zu können. Die Meßvorschrift ist in der Handelsklassenverordnung für Scheinefleisch angegeben.

Ein wesentliches Problem bei der Klassifizierung liegt darin, daß eine sehr hohe Genauigkeit der Messung sichergestellt sein muß, damit der Muskelfleischanteil über die (gesetzlich festgelegte) Verrechnung von Speckmaß und Fleischmaß exakt angebbar ist. Die Bestimmung muß sehr schnell erfolgen, damit das Arbeitspensum von wenigen Bedienungspersonen erledigt werden kann, die darüber hinaus oft nur angelernt sind. Schließlich erfordert die Umgebung, in der die Messung erfolgt, besondere Maßnahmen hinsichtlich Widerstandsfähigkeit und auch Möglichkeit zur Reinigung des Gerätes mit aggressiven Chemikalien. Somit ist die Zuverlässigkeit des Gerätes insgesamt ein ausschlaggebener Faktor für den Verkauf.

Die Klassifizierung wird heute mittels Meßsonden durchgeführt, die entweder auf dem optischen Prinzip arbeiten und einen "Farbvergleich" der durchstochenen Gewebeschichten durchführen oder aber die Leitfähigkeit der verschiedenen Schichten messen und aus den Messungen die gesuchten Maße herleiten.

Der Erfindung liegt die Aufgabe zugrunde, ein Fleischklassifizierungsgerät der eingangs genannten Art dahingehend aus zubilden, daß exakte Meßergebnisse mit hoher Zuverlässigkeit im Dauerbetrieb erreichbar sind.

Diese Aufgabe wird durch die im Kennzeichen des Patentanspruches 1 angegebenen Merkmale gelöst.

Im einzelnen umfaßt das hier aufgezeigte Fleischklassifizierungsgerät eine in das zu untersuchende Prüfstück einstechbare Leitfähigkeitssonde mit einer angekoppelten Auswerteinrichtung zum Feststellen und Anzeigen des Fleisch-/Fettanteils des untersuchten Prüfstückes, wobei die Leitfähigkeitssonde einen Sondenkörper mit einem metallischen Trägerrohr umfaßt, in dessen Oberfläche eine Elektrodenanordnung mit einer sich in Längsrichtung des Sondenkörpers erstreckenden Reihe von Elektroden isoliert zum Trägerrohr eingelassen ist, die mit der Auswerteinrichtung derart verbunden sind, daß bei eingestochener Leitfähigkeitssonde die Widerstands- bzw. die Leitfähigkeitswerte zwischen allen Elektroden und dem Trägerrohr feststellbar und untereinander vergleichbar sind. Es handelt sich also um eine diskontinuierliche Messung, bei welcher jeder Meßpunkt durch die Lage der jeweiligen Elektrode exakt definiert ist. Die Elektroden sind hierbei equidistant voneinander beabstandet, so daß die Errechnung der einzelnen Meßpositionen sehr einfach ist.

Die Leitfähigkeitssonde ist den Anforderungen entsprechend ca. 170 mm lang und weist einen Durchmesser von ca. 10 mm auf. Diese Meßsonde wird gemäß der gesetzlichen Vorschrift 7 cm seitlich der Trennlinie auf der zweit- und drittletzten Rippe von der Schwarte aus senkrecht in die Richtung der Rippen einer frischgeschlachteten Schweinehälfte eingestoßen und durch das Prüfstück hindurchgestoßen. Insgesamt sind 128 Elektroden in einem Abstand von jeweils 1,27 mm voneinander vorgesehen. Mittels der Elektroden wird die Leitfähigkeit des umgebenden Gewebes gemessen, wobei bekanntlich die Leitfähigkeit des Fett- und Bindegewebes etwa um den Faktor 4 geringer ist als die Leitfähigkeit im Muskelfleisch.

Vorzugsweise werden die Elektroden während des Auswertens nacheinander abgetastet, wobei Multiplexer vorgesehen sind, deren Eingänge jeweils mit einer Elektrode und deren Ausgänge mit einer Leitfähigkeitsmeßeinrichtung verbunden sind. Auf diese Weise kann mit nur einer Leitfähigkeitsmeßeinrichtung gearbeitet werden, was den Aufwand selbst dann wesentlich verringert, wenn man diese Leitfähigkeitsmeßeinrichtung hochpräsis mit relativ großem Aufwand gestaltet.

Die Elektrodenanordnung umfaßt einen in das Trägerrohr eingesetzten Kunstharz-Gußkörper, in welchem die Elektroden eingegossen sind. Somit sind die Elektroden zum einen sicher fixiert, was für die Genauigkeit der Messung notwendig ist, zum anderen bilden sie ein beim Zusammenbau des Gerätes leicht handhabbares Einzelteil, das ohne die Gefahr eines Kurzschlusses zwischen einer Elektrode und dem Trägerrohr in dieses einsetzbar ist. Weiterhin ist durch diese Anordnung gewährleistet, daß man das Trägerrohr bei Bedarf austauschen kann.

Die Anordnung wird dann besonders kleinbauend und weist darüber hinaus eine hohe Störsicherheit auf, wenn die Elektroden im wesentlichen direkt an den Eingangsklemmen der Multiplexer angebracht und zusammen mit diesen im Gußkörper eingegossen sind. Dadurch wird eine kompakte Einheit gebildet, die über nur wenige Ausgangsleitungen, nämlich die Stromversorgungen, die Steuerleitungen für die Multiplexer und eine einzige Ausgangsleitung an die übrige Elektronik anschließbar ist.

Vorzugsweise wird die Spitze der Sonde als gesondertes Teil ausgebildet, das auf das Trägerrohr auf- und von diesem abschraubbar ist. Die Konstruktion der Anordnung wird dadurch vereinfacht, wobei weiterhin die Spitze leicht auswechselbar ist, um sie durch eine neue Spitze zu ersetzen bzw. um sie in regelmäßigen Abständen zu schärfen.

Vorzugsweise weist die Spitze eine Schneide auf, die derart gestaltet ist, daß die Reihe von Elektroden an einem Ende des Schnittspaltes zu liegen kommt.

Die Anordnung ist dann besonders gut handhabbar, wenn die Leitfähigkeit der Sonde an einem Gehäuse mit einem Griff zur Bildung einer pistolenförmigen Anordnung befestigt ist. Das Gehäuse weist vorzugsweise eine Anzeigeeinrichtung auf, die von außen sichtbar ist, so daß die Bedienungsperson die Meßwerte ablesen kann. Weiterhin sind am Gehäuse Bedienungstasten zur Auslösung eines Meßvorgangs und zu anderen Zwecken vorgesehen.

Die Auswerteinrichtung wird vorzugsweise so ausgebildet, daß die Leitfähigkeitsmessung über ein Wechselstromsignal erfolgt. Auf diese Weise ist eine sehr exakte Messung möglich, ohne daß die Elektroden bzw. das Sondenrohr einer Korrosion durch Elektrolysevorgänge ausgesetzt sind. Das Wechselstromsignal wird vorzugsweise relativ hochfrequent gemacht, wobei sich eine Frequenz von ca. 20 kHz als geeignet erwiesen hat.

Die Auswerteinrichtung ist weiterhin vorzugsweise so ausgebildet, daß ein Auswertvorgang erst dann auslösbar ist, wenn sich nach dem Durchstechen des Stückes mindestens die spitzennächste Elektrode im Freien bzw. in Umgebungsluft befindet. Hierdurch wird sichergestellt, daß den gesetzlichen Anforderungen (vollständiges Durchstechen des Stückes) Genüge getan wird, wobei dies durch Abtasten zumindest der spitzennächsten Elektrode, vorzugsweise aber durch Abtasten der spitzennächsten fünf Elektroden geschieht, was die Sicherheit der Messung wesentlich erhöht.

Vorzugsweise wird ein Auswertvorgang nur dann als verwertbar angenommen bzw. kann nur dann ausgelöst werden, wenn keine störende Relativbewegung zwischen der Leitfähigkeitssonde und dem Prüfstück vorliegt. Dies kann dadurch überprüft werden, daß man die Leitwerte an mehreren Elektroden mehrmals (mindestens zweimal) hintereinander überprüft und die gewonnenen Werte miteinander vergleicht. Wenn diese zumindest innerhalb eines vorbestimmten Toleranzmaßes übereinstimmen, so kann davon ausgegangen werden, daß die Sonde im Prüfstück ruht. Vorteilhafterweise werden hierbei sämtliche Elektroden mehrmals (mindestens zweimal) hintereinander abgetastet, so daß bei übereinstimmenden Wertreihen (die Sonde ruht) beide Meßreihen unter Bildung eines Mittelwertes zur Auswertung herangezogen werden können, was die Genauigkeit des Meßergebnisses weiter erhöht.

Bei einer ersten Ausführungsform der Erfindung kann man die Einstichtiefe bzw. die Lage der Schwarte auf der Meßsonde und damit die Relativposition sämtlicher Elektroden zur Meßsonde auf rein elektronischem Weg über eine Auswertung der Leitfähigkeitsmessung ermitteln. Hierbei wird davon ausgegangen, daß an beiden Enden der Meßsonde Elektroden in Umgebungsluft liegen. Vorzugsweise wird jedoch die Sonde bis zu einem Anschlag, insbesondere bis zum Gehäuse eingestochen, und die Auswertung nur dann vorgenommen, wenn sich die gehäusenächste Elektrode im Probestück, also nicht in Luft befindet. Zum einen ist diese Art der Messung einfacher, da ein Längenmeßpunkt bereits mechanisch festgelegt ist, zum anderen auch sicherer.

Um die Auflösung bei der Messung zu erhöhen, wird vorzugsweise ein Vergleich der Leitfähigkeitsmeßwerte an den im Grenzbereich liegenden Elektroden mit festgestellten Leitfähigkeitswerten vorgenommen und zwischen den Meßwerten derart interpoliert, daß eine gewisse Anzahl von Zwischen-Meßwerten herleitbar ist.

Vorzugsweise ist die Auswerteinrichtung weiterhin derart ausgebildet, daß der Absolutbetrag des an mindestens einer Elektrode gemessenen Leitfähigkeitswertes feststell- und ausgebbar ist, welcher der Leitfähigkeit des durchstochenen Muskelfleisches entspricht. Anhand dieses gewonnenen Leitfähigkeitswertes ist es möglich, den Wassergehalt des untersuchten Muskelfleisches zu bestimmen, der dann einen Hinweis für die weitere mögliche Verarbeitung des Fleisches gibt. Es werden somit bei dieser Ausführungsform der Erfindung zwei für den Käufer wesentliche Parameter zugänglich, zum einen der Muskelfleischanteil des untersuchten Stückes, zum anderen dessen Qualität.

Die Feststellung des Muskelfleisch-Leitfähigkeitswertes erfolgt hierbei vorzugsweise derart, daß zwar über mehrere Leitwerte entsprechend mehreren Elektroden gemittelt wird, diejenigen Meßwerte aber bei der Mittelung außer acht gelassen werden, die aufgrund starker Abweichung offensichtlich von Elektroden stammen, die sich in Bindegewebsschichten befinden, welche das Muskelfleisch durchziehen. Dies kann über an sich bekannte statistische Methoden der Stichprobenauswertung geschehen, wie sie z. B. in Lothar Sachs: Statistische Methoden, Springer 1979 beschrieben sind.

Weitere erfindungswesentliche Merkmale ergeben sich aus den Unteransprüchen und der nachfolgenden Beschreibung einer bevorzugten Ausführungsform der Erfindung, die anhand von Abbildungen näher erläutert wird. Hierbei zeigen:

Fig. 1 eine teilgeschnittene Seitenansicht einer Ausführungsform des Fleischklassifizierungsgerätes,

Fig. 2 eine vergrößerte Teilansicht des mit II bezeichneten Ausschnittes aus Fig. 1;

Fig. 3 eine Seitenansicht der Spitze des Gerätes nach Fig. 1;

Fig. 4 eine Ansicht in Richtung des Pfeiles IV aus Fig. 1;

Fig. 5 ein schematisiertes Blockschaltbildung des erfindungsgemäßen Gerätes, und

Fig. 6 - 8 schematisierte Darstellungen zur Erläuterung des Interpoliervorganges beim Auswerten von Leitfähigkeitsmeßwerten.

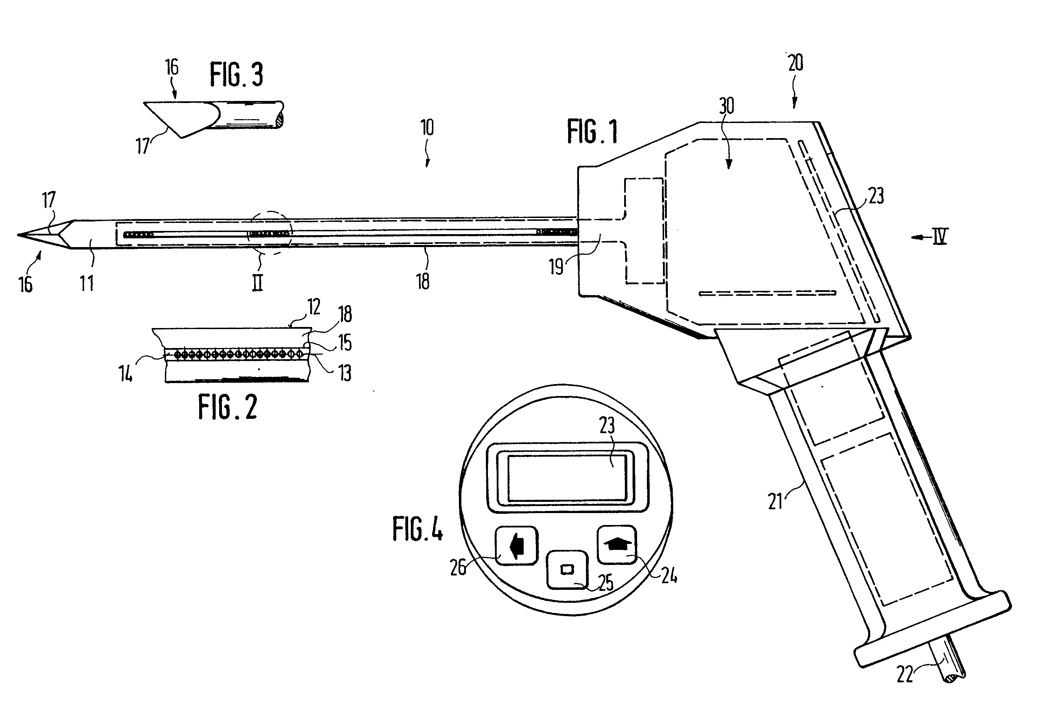

Wie aus den Fig. 1 bis 3 hervorgeht, umfaßt die hier gezeigte Ausführungsform des erfindungsgemäßen Fleischklassifizierungsgerätes eine Leitfähigkeitssonde 10, die an einem pistolenartig geformten Gehäuse 20 angebracht ist. Die Leitfähigkeitssonde 10 umfaßt einen stabförmigen Sondenkörper 11, der an seinem, dem Gehäuse 20 abgewandten Ende mit einer Spitze 16 versehen ist. Die Spitze 16 weist eine schrägliegende Schneide 17 auf und ist über ein nichtgezeigtes Gewinde auf ein Trägerrohr 18 aufgeschraubt.

Das Trägerrohr 18 weist im wesentlichen über seine gesamte, aus dem Gehäuse 20 ragende Länge ein schlitzförmiges Fenster 15 auf.

Im Fenster 15 sind Elektroden 13 in Isoliermaterial 14 eingebettet derart angebracht, daß ihre Oberflächen mit der Oberfläche 12 des Trägerrohres 18 bündig abschließen. Somit ist die Leitfähigkeitssonde 10 auf ihrer Oberfläche im wesentlichen durchgängig glatt ausgebildet, so daß keine Rückstände anhaften können. Die Materialien sind so gewählt, daß die Anordnung auch mit aggressiven Reinigungsmitteln und bei höheren Temperaturen gereinigt werden kann, wobei das Sondenrohr 18 vorzugsweise aus V2A-Stahl besteht.

Das Gehäuse 20 weist einen Griff 21 auf, an welchem das Gerät zum Einstechen angefaßt werden kann. Im Gehäuse 20 ist eine weiter unten näher beschriebene Auswerteinrichtung 30 vorgesehen, deren Auswertergebnisse sowohl auf einer, am Gehäuse-Hinterende angebrachten LCD-Anzeige 23 (siehe Fig. 4) angezeigt als auch über ein Kabel 22 zu einer externen Anzeige- und Protokolliereinrichtung (Drucker) übermitteln werden können. Weiterhin sind in der Nähe der Anzeige 23 Bedienungstasten 24, 25 und 26 angebracht, über welche Protokolldaten eingebbar, der Meßvorgang auslösbar und bestimmte Betriebsarten wählbar sind. Die auf einer Anzeigeplatine angeordnete Anzeige 23 mit den Bedienungstasten 24 - 26 ist über eine (nichtgezeigte) Folie nach außen abgedeckt, durch die zum einen eine sichere Abdichtung und zum anderen auch ein unbefugtes Eingreifen in das Gerät verhindern werden können.

Im Griff 21 befindet sich zur Stromversorgung der Auswerteinrichtung 30 ein Akkumulator, der über eine dazugehörige Ladeschaltung von einem externen Ladegerät geladen werden kann, das über das Kabel 22 angekoppelt ist. Das Gehäuse und der Griff sind vorzugsweise aus Aluminiumguß (oberflächengeschützt) gefertigt.

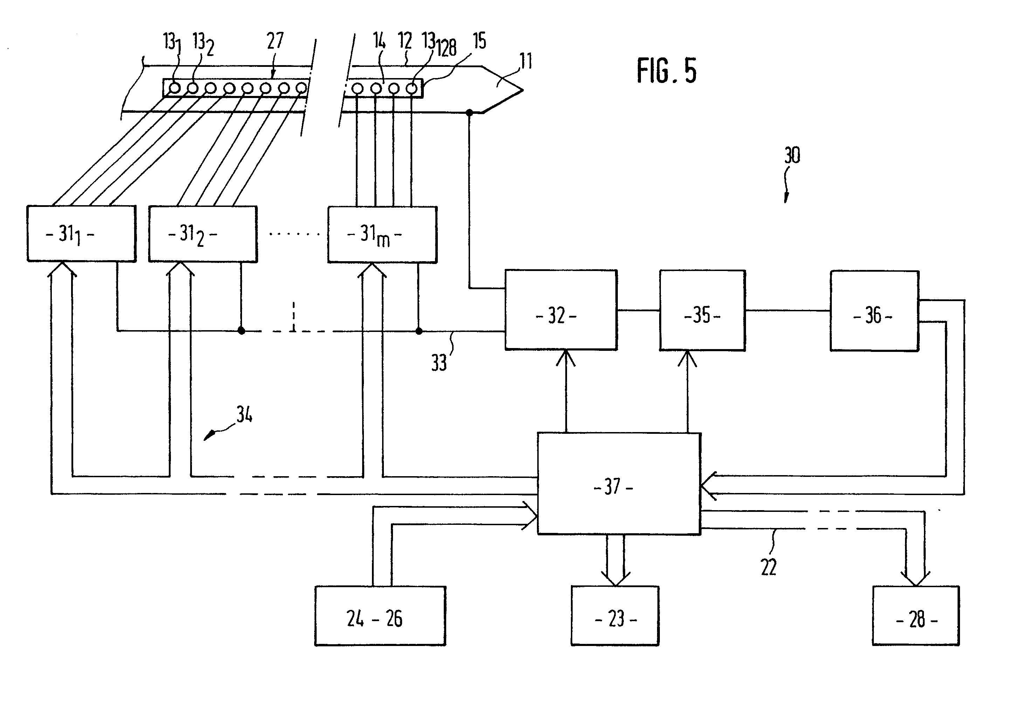

Im folgenden wird der Aufbau der Elektronik des hier gezeigten Fleischklassifizierungsgerätes anhand von Fig. 5 erläutert.

Jede der insgesamt 128 Elektroden 13 ist mit einem Eingang eines Multiplexers 31 verbunden. In Fig. 5 sind aus Gründen der Darstellung Multiplexer mit lediglich vier Eingängen gezeigt, jedoch werden vorzugsweise solche mit mehr Eingängen (insbesondere mit acht Eingängen) verwendet.

Die Multiplexer 31 sind auf eine gemeinsame Ausgangsleitung 33 geführt und können über eine Steuerleitung 34 derart angesteuert werden, daß die Elektroden $13_1$ bis $13_{128}$ der Reihe nach mit der Ausgangsleitung 33 verbunden werden. Die Steuerung der Multiplexer 31 erfolgt hierbei über eine Steuereinheit 37.

Die Ausgangsleitung 33 steht mit einer Eingangsklemme einer Leitfähigkeitsmeßeinrichtung 32 in Verbindung, deren andere Eingangsklemme mit dem metallischen Sondenkörper 11 in Verbindung steht. Die Leitfähigkeitsmeßeinrichtung 32 ist hierbei derart ausgebildet, daß der Widerstand zwischen der jeweils von den Multiplexern 31 mit der Ausgangsleitung 33 verbundenen Elektrode 13 und dem Sondenkörper 11 über die Speisung mit einem 20 kHz-Wechselstromsignal gemessen wird. Aus diesem Widerstand wird dann der Kehrwert, also die Leitfähigkeit hergeleitet.

Der Ausgang der Leitfähigkeitsmeßeinrichtung 32 ist mit einer Schaltung 35 verbunden, in welcher das Wechselstromsignal derart demoduliert wird, daß sein Spitzenwert vorliegt, der dann über eine sample-and-hold-Schaltung gehalten und in einem nachfolgenden A/D-Wandler 36 in einen entsprechenden Digitalwert gewandelt wird. Der A/D-Wandler 36 ist mit seinen Digitalausgängen mit einem entsprechenden Eingang der Steuereinheit 37 verbunden. Weiterhin ist die Steuereinheit 37 über Steuerleitungen mit der Leitfähigkeitsmeßeinrichtung 32 und der Schaltung 35 zu deren Steuerung verbunden.

Die eingangs erwähnten Bedienungstasten 24 - 26 sowie die Anzeige 23 sind ebenfalls mit der Steuereinheit 37 verbunden. Schließlich ist eine externe Verarbeitungseinrichtung 28, z. B. ein Drucker, über das Kabel 22 mit der Steuereinheit 37 verbunden.

Während sich die Schaltungen 32 und 35 - 37 auf entsprechenden Platinen innerhalb des Gehäuses 20 befinden, sind die Multiplexer 31 im Trägerrohr 18 angeordnet. Hierzu sind die Elektroden 13 im wesentlichen direkt mit den Eingangskontakten der Multiplexer-IC's verbunden, wobei die so entstehende Anordnung mit Kunstharz vergossen ist, so daß ein kompakter Gußkörper 19 entsteht (siehe Fig. 1). Neben den Stromversorgungen für die Multiplexer 31 sind somit lediglich die Steuerleitung 34 und die Ausgangsleitung 33 noch im Trägerrohr 18 unterzubringen, was eine große Platzersparnis bei gleichzeitig hoher Störsicherheit mit sich bringt. Insbesondere sind die Fehler, die sich bei der Leitfähigkeitsmessung durch lange Zuleitungskabel unvermeidbar ergeben, wesentlich verringert. Es sei hierbei noch darauf verwiesen, daß die Verbindung zwischen der Leitfähigkeitsmeßeinrichtung 32 und dem Sondenkörper 11 an dessen Spitze eingezeichnet ist, jedoch bei der tatsächlichen Ausführung die Verbindung direkt am Gehäuse angebracht, das gleichzeitig einen virtuellen Massepunkt bildet.

Die Verbindung zwischen der so ausgebildeten Auswerteinrichtung 30 und dem Sondenkörper 11 mit den darin enthaltenen Multiplexern 31 ist als Steckverbindung ausgeführt, so daß ein Austausch der Sondenspitze mit darin enthaltenen Elektronikteilen leicht möglich ist.

Im folgenden wird das erfindungsgemäße Verfahren zum Betrieb des hier gezeigten Fleischklassifizierungsgerätes erläutert.

Bei der Herstellung des Gerätes wird zur stets genauen Ermittlung der Leitfähigkeit des untersuchten Probestückes die Sonde mit einer Kalibrierlösung mit definierter Leitfähigkeit eingestellt. Die unterschiedlichen Elektroden-Konstanten bzw. Empfindlichkeiten der einzelnen "Leitfähigkeitsmeßzellen" werden hierdurch ermittelt und als Korrekturgrößen gespeichert. Bei allen späteren Messungen können somit diese einmal festgelegten Korrekturgrößen zur Ermittlung von Absolutwerten der Leitfähigkeit des Fleisches bzw. des Fettgewebes verwendet werden.

Bei jeder aktuellen Messung wird die Sonde in das Probestück von der Schwarte her soweit eingestochen, bis das Gehäuse 20 auf der Schwarte aufliegt und die Sondenspitze das Stück durchstochen hat. Wenn nun die Bedienungsperson durch Betätigung der Taste 25 eine Messung auslösen will, so werden zunächst die spitzennächsten fünf Meßpunkte abgetastet. Wenn die gemessenen Leitwerte gegen Null gehen, d. h. also die entsprechenden Elektroden sich in Umgebungsluft befinden, so ist sichergestellt, daß entsprechend der Verordnung die vorgeschriebene Meßstrecke im Probestück vollständig durchlaufen ist. Erst dann kann von der Bedienungsperson die Messung tatsächlich ausgelöst werden.

Nach Auslösung der Messung durch die Bedienungsperson führt die Steuereinheit 37 zwei Meßzyklen aus, die jeweils ca. 0,6 s dauern. Bei jedem Meßzyklus werden alle 128 Elektroden der Reihe nach abgetastet und die entsprechenden Leitwerte gespeichert. Nun werden die gespeicherten Meßwerte jeder einzelnen Elektrode aus beiden Meßzyklen miteinander so verglichen, daß der Meßwert der ersten Elektrode aus dem ersten Meßzyklus mit dem Meßwert derselben Elektrode aus dem zweiten Meßzyklus verglichen wird. Wenn die Abweichung etwa 10% der Leitfähigkeit nicht überschreitet, so werden beide Meßreihen (unter Bildung eines Mittelwertes) zur Auswertung herangezogen. Wenn die Meßwerte zu stark voneinander abweichen, so wird daraus geschlossen, daß sich die Sonde während des Abtastvorganges noch relativ zum Probestück bewegt hat, so daß dann beide Meßreihen verworfen und ein neuer Meßvorgang gestartet wird.

Nach Durchführung von (zwei) gültigen Meßzyklen werden jeweils die Gruppen von aufeinanderfolgenden Elektroden ermittelt, an denen jeweils im wesentlichen gleiche Leitfähigkeiten gemessen wurden. Da jedes Muskelgewebe von Bindegewebe umschlossen ist, kann durch die damit gegebene eindeutige Trennung von umgebendem Gewebe und die daraus folgende, sich sprunghaft ändernde Leitfähigkeit eindeutig zwischen Muskelfleisch und Fett- oder Zwischenrippengewebe unterschieden werden.

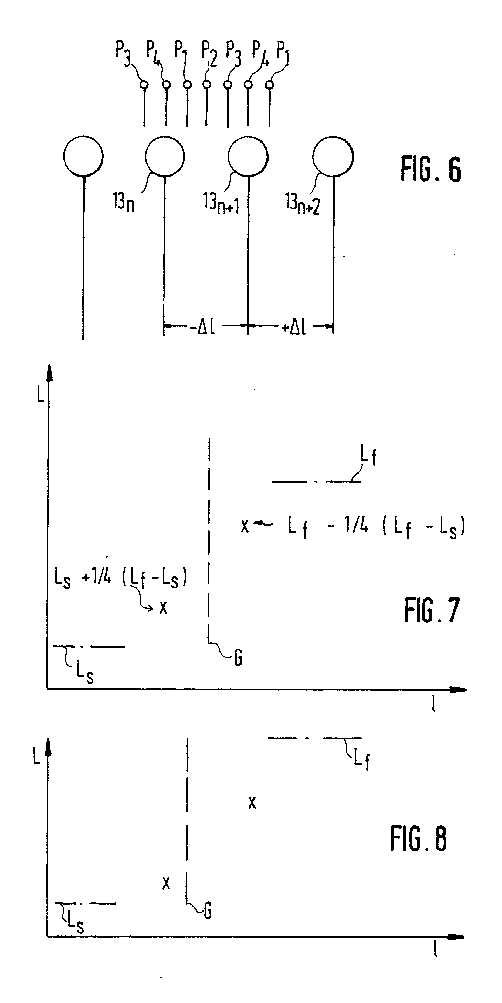

Um eine möglichst hohe Genauigkeit bei der Messung des Fleischanteils zu erreichen, ist es notwendig, die Grenze zwischen Fleisch- und Fettgewebe und auch zwischen Fleisch- und Zwischenrippengewebe sehr genau zu bestimmten. Diese Bestimmung erfolgt nun vorzugsweise mit einer gegenüber dem vorgegebenen Elektrodenabstand erhöhten Genauigkeit, wie dies im folgenden anhand der Fig. 6 bis 8 erläutert wird.

In Fig. 6 sind vier Elektroden schematisiert eingezeichnet und darüberliegend Punkte P1 - P4, welche eine feinere MeßUnterteilung bilden. Die einzelnen Abstände zwischen den Elektroden sind in Fig. 6 mit Al bezeichnet. In den Fig. 7 und 8 sind mit 1 die Meßstrecke, mit L die Leitfähigkeit, mit G die Gewebegrenze, mit Lf die Leitfähigkeit im Fleisch, mit Ls die Leitfähigkeit im Speck (Fett)-Gewebe und mit x die tatsächlichen Meßwerte bezeichnet.

Im folgenden wird nun angenommen, daß die zu ermittelnde Gewebegrenze zwischen der Elektrode 13n und der Elektrode 13 (n + 1 liegt. Die Reichweite des elektrischen Feldes einer Elektrode beträgt etwa ± $\Delta$1, d. h. entspricht etwa dem Elektrodenabstand zu beiden Seiten einer Meßelektrode.

Wenn nun, wie in Fig. 7 gezeigt, die Gewebegrenze genau in der Mitte zwischen den Elektroden 13n und 13 (n + 1 ) liegt, so werden die Meßwerte an diesen beiden Elektroden beeinflußt. Im wesentlichen werden dann die Meßwerte an diesen beiden Elektroden um ein Viertel des Leitfähigkeitsunterschiedes, d. h. um ein Viertel (Lf - Ls) an der Elektrode 13n angehoben bzw. an der Zelle 13 (n + 1 ) abgesenkt. Durch diese symmetrische Lage der Leitfähigkeitsunterschiede kann somit darauf geschlossen werden, daß die Gewebegrenze G genau in der Mitte zwischen den Elektroden liegt. Dies entspricht dann einem Meßpunkt P2, wie er in Fig. 6 gezeigt ist.

Wenn die Gewebegrenze in der ersten Hälfte des Bereiches zwischen den Elektroden 13n und 13 (n + 1) liegt, also beim Punkt P1 (Fig. 6) bzw. bei der in Fig. 8 gezeigten Position, so muß die an der Elektrode 13n gemessene Leitfähigkeit größer als 1/2 (Ls + Lf) sein, während die an der Elektrode 13 (n + 1 ) gemessene Leitfähigkeit um einen geringeren Betrag angehoben sein muß, als dies beim zuvor geschilderten Fall nach Fig. 7 der Fall ist. Es kann also durch eine entsprechende Interpolierung zwischen den Meßwerten zumindest entschieden werden, ob sich die Gewebegrenze innerhalb bestimmter Bereiche zwischen einzelnen Elektroden befindet. Die hierdurch erzielbare Auflösung beträgt aufgrund der Festlegung gemäß den oben beschriebenen Kriterien etwa ± 1/4 x Δ1, also bei einem Elektrodenabstand von 1,27 mm etwa 0,32 mm.

Unter Zugrundelegung der gesetzlich festgelegten Rechenregel zur Bestimmung des Fleischanteils läßt sich dann errechnen, daß der mit dem hier gezeigten Fleischklassifizierungsgerät erzielbare Meßfehler nur etwa 0,3 Muskelfleischprozent beträgt. Dies ergibt bei einem durchschnittlichen Fleischanteil von ca. 58 % einen relativen Fehler von nur ca. 0,5%.

Wie eingangs erwähnt, wird mit dem erfindungsgemäßen Gerät auch gleichzeitig die Leitfähigkeit des Muskelgewebes ermit telt, da diese eine Aussage über den Anteil an freiem Zellwasser zuläßt, was wiederum für die Fleischqualität und somit für die spätere Verarbeitung mitentscheidend ist. Dies geschieht dadurch, daß zunächst bestimmt wird, welche Elektroden sich tatsächlich in Muskelgewebe befinden, so daß ein Einfluß von Meßwerten, die aus Fettgewebe oder Zwischenrippengewebe stammen, ausgeschlossen werden kann. Weiterhin wird dann bestimmt wie die Verteilung der einzelnen Meßwerte aussieht, so daß "Ausreißer" feststellbar und bei der nachfolgenden Mittelwertsbildung über die im Fleisch befindlichen Elektroden (bzw. die zugehörigen Leitfähigkeitsmeßwerte) unterdrückbar sind. Dies kann z. B. durch an sich bekannte statistische Methoden erfolgen. So z. B. ist es möglich, ein Histogramm über die (Muskelfleisch-)Leitfähigkeitsmeßwerte zu bilden und nur diejenigen Meßwerte zur Mittelwertsbildung heranzuziehen, welche mit einer Häufigkeit von 80 oder 90 % auftreten. Auf diese Weise kann sichergestellt werden, daß diejenigen Leitfähigkeitsmeßwerte keinen Einfluß in das Meßergebnis finden, welche von Elektroden stammen, die sich in dünnen Bindegewebs- oder Fettschichten befinden, welche das Muskelfleisch durchziehen.

Aus Obigem geht hervor, daß die Erfindung sich auch auf vielfältige Abwandlungen bezieht, wobei es insbesondere auf die Elektrodenanordnung ankommt. Es ist beispielsweise möglich, anstelle eines metallischen Trägerrohrs ein solches aus nichtleitendem Material zu verwenden, wobei dann die Leitfähigkeitsmessung nicht zwischen einer Einzelelektrode und dem Trägerrohr, sondern zwischen einer Einzelelektrode und sämtlichen anderen, parallelgeschalteten Elektroden erfolgt.

**Bezugszeichenliste:**

10 Leitfähigkeitssonde
11 Sondenkörper
12 Oberfläche
13 Elektroden
14 Isoliermaterial
15 Fenster
16 Spitze
17 Schneide
18 Trägerrohr
19 Gußkörper
20 Gehäuse
21 Griff
22 Kabel
23 Anzeige
24 Bedienungstaste
25 Bedienungstaste
26 Bedienungstaste
27 Elektrodenanordnung
28 Externe Verarbeitungseinheit
30 Auswerteinrichtung
31 Multiplexer
32 Leitfähigkeitsmeßeinrichtung
33 Ausgangsleitung
34 Steuerleitung
35 Demodulator; Sample and Hold-Schaltung
36 A/D-Wandler
37 Steuereinheit

**Ansprüche**

1. Fleischklassifizierungsgerät mit einer Sonde, mit einem Sondenkörper, der ein metallisches Trägerrohr umfaßt, in dessen Oberfläche eine Anordnung mit einer sich in Längsrichtung des Sondenkörpers erstreckenden Reihe von Sensoren eingelassen ist, die mit einer Auswerteinrichtung derart verbunden sind, daß bei eingestochener Sonde die Meßwerte aller Sensoren feststellbar und untereinander vergleichbar sind, wobei die Sonde als Leitfähigkeitssonde ausgebildet ist, die Sensoren eine Elektrodenanordnung mit einer Reihe von Elektroden umfassen, die isoliert zum Trägerrohr angeordnet sind, und die Reihe von Elektroden über im wesentlichen die gesamte Sondenlänge vorgesehen ist;

**dadurch gekennzeichnet,**

daß die Auswerteinrichtung (30) derart ausgebildet ist, daß bei eingestochener Sonde (19) die Widerstandswerte und damit die Leitfähigkeitswerte zwischen allen Elektroden (13) und dem Trägerrohr (18) feststellbar und untereinander vergleichbar sind.

2. Gerät nach Anspruch 1,

**dadurch gekennzeichnet,**

daß über Steuerleitungen (34) steuerbare Multiplexer (31) Svorgesehen sind, deren Vielzahl von Eingängen jeweils mit einer Elektrode (13) und deren Ausgänge über mindestens eine Ausgangsleitung (33) mit einer Leitfähigkeitsmeßeinrichtung (32) verbunden sind.

3. Gerät nach einem der vorhergehenden Ansprüche,

**dadurch gekennzeichnet,**

daß die Elektrodenanordnung (27) einen in das Trägerrohr (18) einsetzbaren Kunstharz-Gußkörper (19) umfaßt, in welchen die Elektroden (13) eingegossen sind.

4. Gerät nach den Ansprüchen 2 und 3,

**dadurch gekennzeichnet,**

daß die Elektroden (13) im wesentlichen direkt an Eingangskontakten der als integrierte Bausteine ausgeführten Multiplexer (31) angebracht und zusammen mit diesen im Gußkörper (19) eingegossen sind.

5. Gerät nach einem der vorhergehenden Ansprüche,

**dadurch gekennzeichnet,**

daß das Trägerrohr (18) mit einer auf-/abschraubbaren Spitze (16) zum Einstechen versehen ist.

6. Gerät nach einem der vorhergehenden Ansprüche,

**dadurch gekennzeichnet,**

daß die Leitfähigkeitssonde (10) an einem Gehäuse (20) mit einem Griff (21) zur Bildung einer pistolenförmigen Anordnung befestigt ist.

7. Gerät nach Anspruch 6,

**dadurch gekennzeichnet,**

daß im Gehäuse (20) eine Anzeigeeinrichtung (23) von außen sichtbar vorgesehen ist.

8. Gerät nach einem der Ansprüche 6 oder 7,

**dadurch gekennzeichnet,**

daß am Gehäuse (20) mindestens eine Bedienungstaste (24-26) zur Auslösung eines Meßvorganges und/oder Eingabe von Protokolldaten und/oder Eingabe von Betriebsarten vorgesehen ist.

9. Gerät nach einem der vorhergehenden Ansprüche,

**dadurch gekennzeichnet,**

daß die Auswerteinrichtung (30) derart ausgebildet ist, daß die Leitfähigkeitsmessung über ein Wechselstromsignal erfolgt.

10. Gerät nach Anspruch 9,

**dadurch gekennzeichnet,**

daß das Wechselstromsignal eine Frequenz zwischen 10 und 50, vorzugsweise von etwa 20 kHz aufweist.

11. Verfahren zum Feststellen und Anzeigen des Fleisch/Fettanteils eines zu untersuchenden Prüfstücks, wobei eine Sonde in das Prüfstück eingestochen wird, die Sonde einen Sondenkörper aufweist, der ein metallisches Trägerrohr umfaßt, in dessen Oberfläche eine Anordnung mit einer sich in Längsrichtung des Sondenkörpers erstreckenden Reihe von Sensoren eingelassen ist, die mit einer Auswerteinrichtung derart verbunden sind, daß bei eingestochener Sonde die Meßwerte aller Sensoren feststellbar und untereinander vergleichbar sind, wobei die Sonde als Leitfähigkeitssonde ausgebildet ist, die Sensoren eine Elektrodenanordnung mit einer Reihe von Elektroden umfassen, die isoliert zum Trägerrohr angeordnet sind und die Reihe von Elektroden über im wesentlichen die gesamte Sondenlänge vorgesehen ist,

**dadurch gekennzeichnet,**

daß bei eingestochener Sonde die Widerstandswerte und damit die Leitfähigkeitswerte zwischen allen Elektroden und dem Trägerrohr festgestellt und untereinander verglichen werden.

12. Verfahren nach Anspruch 11,

**dadurch gekennzeichnet,**

daß ein Auswertvorgang erst dann auslösbar ist, wenn sich nach dem Durchstechen des Probestückes mindestens die spitzennächste Eletrode (13) im Freien bzw. in Luft befindet.

13. Verfahren nach einem der Ansprüche 11 oder 12,

**dadurch gekennzeichnet,**

daß ein Auswertvorgang nur dann als verwertbar angenommen wird, wenn keine störende Relativbewegung zwischen Leitfähigkeitssonde und Prüfstück vorliegt.

14. Verfahren nach Anspruch 13,

**dadurch gekennzeichnet,**

daß Leitwerte an mehreren Eletroden (13) mindestens zweimal hintereinander abgetastet und miteinander verglichen werden, und ein Auswertvorgang nur dann als verwertbar angenommen wird, wenn die jeweiligen Leitwerte an den Elektroden (13) aus den verschiedenen Abtastvorgängen um weniger als ein vorgegebenes Toleranzmaß (10 %) voneinander abweichen.

15. Verfahren nach Anspruch 14,
**dadurch gekennzeichnet,**
daß sämtliche Elektroden (13) abgetastet werden und verwertbare Auswertungen die Mittelwerte der Einzel-Leitfähigkeitswerte umfassen.

16. Verfahren nach einem der Ansprüche 11 - 15,
**dadurch gekennzeichnet,**
daß ein Auswertvorgang nur dann als verwertbar angenommen wird, wenn die Sonde (10) bis zu einer vorbestimmten Tiefe (Gehäuseansohlag) eingestochen ist.

17. Verfahren nach Anspruch 16,
**dadurch gekennzeichnet,**
daß ein Auswertvorgang nur dann als verwertbar angenommen wird, wenn sich die gehäusenächste Elektrode (13) im Probestück, also nicht in Luft befindet.

18. Verfahren nach einem der Ansprüche 12 - 17,
**dadurch gekennzeichnet,**
daß bei nichtauslösbaren oder verwertbaren Auswertvorgängen ein entsprechendes Anzeigesignal abgegeben wird.

19. Verfahren nach einem der Ansprüche 11 - 18,
**dadurch gekennzeichnet,**
daß eine Speck-Leitfähigkeit (Ls) und eine (Muskel-) Fleischleitfähigkeit (Lf) feststellbar und aus einem Vergleich der an einzelnen Elektroden (13) gemessenen Werte mit den festgestellten Leitfähigkeitswerten (Ls, Lf) ein Speckmaß (S) ein Fleischmaß (F) und ein Muskelfleischanteil (MF) errechnet und angezeigt werden.

20. Verfahren nach Anspruch 18,
**dadurch gekennzeichnet,**
daß die Lage der Grenze zwischen Muskelfleisch und Speck durch einen Vergleich der Leitfähigkeitswerte an den im Grenzbereich liegenden Elektroden (13) mit den festgestellten Leitfähigkeitsmeßwerten und eine Interpolation der Werte bestimmt wird.

21. Verfahren nach einem der Ansprüche 11 - 20,
**dadurch gekennzeichnet,**
daß der Absolutbetrag des an mindestens einer Elektrode (13) gemessenen Leitfähigkeitswertes feststell- und abgebbar ist, welcher der Leitfähigkeit des durchstochenen Muskelfleisches entspricht.

22. Verfahren nach Anspruch 21,
**dadurch gekennzeichnet,**
daß zunächst festgestellt wird, welche Elektroden (13) sich im Muskelfleisch befinden, und daß dann ein Mittelwert mindestens über eine Teilanzahl der Leifähigkeitsmeßwerte an diesen Elektroden (13) gebildet und abgegeben wird.

23. Verfahren nach Anspruch 22,
**dadurch gekennzeichnet,**
daß nach Feststellung der Grenzen des Muskelfleisches die Leitfähigkeitswerte der Elektroden (13) innerhalb des Muskelfleisches festgestellt und extrem abweichende Werte bei einer nachfolgenden Mittelwertsbildung außer acht gelassen werden.

24. Verfahren nach einem der Ansprüche 11 - 23,
**dadurch gekennzeichnet,**
daß ein Protokoll über die durchgeführten Messungen bzw. Auswertungen an ein externes Aufzeichnungsgerät (28) ausgebbar ist.

FIG.1

FIG.2

FIG.3

FIG.4

FIG. 5

EP 0 411 443 A2

$P_3$   $P_4$   $P_1$   $P_2$   $P_3$   $P_4$   $P_1$

FIG. 6

$13_n$   $13_{n+1}$   $13_{n+2}$

$-\Delta l$   $+\Delta l$

$L$

$L_f$

$x \sim L_f - 1/4\ (L_f - L_s)$

$L_s + 1/4\ (L_f - L_s)$
$\rightarrow x$

FIG. 7

$L_s$

$G$

$l$

$L$

$L_f$

$x$

FIG. 8

$x$

$G$

$L_s$

$l$